# EUROPEAN PATENT APPLICATION

(11) **EP 1 894 517 A2**
(43) Date of publication of application: **05.03.2008**
(21) Application number: 07016967.7
(22) Date of filing: 30.08.2007
(51) Int. Cl.: A61B 1/07, A61B 1/04

(54) **Endoscopic device with temperature based light source control**

(30) Priority: 30.08.2006 US 512918
(71) Applicant: Karl Storz Endovision, Inc., Charlton, MA 01507 (US)
(72) Inventor: Landry, Dana J., Sturbridge MA 01566 (US)
(74) Representative: Weller, Wolfgang

(57) **Abstract**

An endoscopic system includes an endoscopic device having a light guide passing therethrough to a distal end thereof, a light source in communication with the endoscopic device, the light source transmitting illumination light to the light guide, a light source controller in communication with the light source, the light source controller controlling an intensity of the illumination light transmitted to the light guide, and at least one temperature sensor, at least a portion of which is carried by the endoscopic device. The temperature sensor senses a temperature of at least a portion of the endoscopic device and produces a signal indicative of the sensed temperature, the signal being transmitted to the light source controller. The light source controller varies the intensity of the illumination light transmitted to the light guide based at least in part upon the signal indicative of the sensed temperature.

## Description

### Field of the Invention

This present invention relates generally to an endoscopic system including a light source for supplying illumination light to an endoscopic device, and more particularly to such an endoscopic system in which the light source is automatically controlled based upon temperature related feedback.

### Background of the Invention

The imaging of body surfaces through endoscopes is well known within the medical and veterinary fields. Typically, this involves inserting an endoscope into a body cavity and directing an intense light source output through the endoscope to illuminate body tissue. Light reflected by the body tissue then is guided along an optical path either to an eyepiece for direct viewing of the tissue (in the case of a conventional endoscope) or to an image sensor to generate a video image of the tissue (in the case of an electronic endoscope).

The illumination light emanating from the light source is propagated to the distal part of the insertion unit portion of the endoscope over a light guide or the like. The illumination light is irradiated to an object region, such as a lesion or other body tissue, via an illumination optical system through the distal part.

In the case of conventional endoscopes, an image of the object region is formed by an objective lens located in the distal part of the insertion unit. An optical image is transmitted to an eyepiece unit by means of an optical transmission means. An eyepiece optical system then enables the optical image to be viewed. The optical transmission means varies depending on the usage or purpose of use. For example, fiber bundles are typically used in conjunction with flexible endoscopes, while relay lens systems are typically used in conjunction with rigid endoscopes.

In electronic endoscopes, which include a solid-state imaging device, such as a CCD, in the distal part of an insertion unit, an optical image is formed on the image formation surface of the CCD via an objective lens. The CCD photoelectrically converts the optical images so as to provide image information in the form of electrical signals. The image information is subjected to various kinds of image processing, and thus a desired image of an object region is displayed on a monitor or the like.

A disadvantage of traditional endoscopic devices, whether conventional or electronic, is that they can become hot during use due to the high amount of light energy passing therethrough required to illuminate the area or cavity of observation. This is especially true in the case of metal bodied, rigid endoscopes, but excessive heating can also occur in connection with semi-rigid and flexible endoscopic devices. It is desired that the temperature of any exposed part of endoscopic devices not exceed 50 °C, or that allowed by applicable standard.

It is well-understood that the temperature of endoscopic devices can be altered by adjusting the intensity of light passing therethrough. Traditionally, this was accomplished by manually adjusting a dial or the like on the light source controller to either increase or decrease the intensity of the illumination light. This technique, however, is problematic in that it distracts the operator's attention away from the medical procedure being performed. Moreover, it is difficult to know precisely how much the intensity of the light should be varied in order to ensure that the threshold temperature is not exceeded, while at the same time ensuring that the intensity of the illumination light is maintained as high as is safely possible to enhance viewing. An automated control solution based upon sensed temperature would be far more desirable.

While there are known prior art systems which provide some level of automated control over the light source, such as those that turn off the light source after a predetermined duration of time (U.S. Patent No. 4,963,960), those that turn off the light source when the light source is not directed at a surface (U.S. Patent No. 6,511,422), and those that set the light intensity based upon an image signal produced by an imaging unit, such as a CCD, in order to optimize the captured image (U.S. Patent Nos. 5,131,381 and 5,957,834), the applicant is not aware of any systems that control the intensity of the illumination light based upon a sensed temperature of some portion of the endoscopic device.

One of the reasons for this may be that traditional types of temperature sensors are not appropriate for use in measuring temperature along an endoscopic device. For example, while the application of thermocouples for measuring temperature is well known, such devices could not be effectively used in connection with measuring temperature along an endoscope for a number of reasons. More specifically, thermocouples are typically relatively large with respect to the available volume within the endoscopic device, thermocouples are electrical devices that create voltage, and therefore may compromise patient safety, and thermocouples require a mechanical wire connection, which may be problematic since endoscopic devices typically must be autoclaved.

What is desired, therefore, is an endoscopic system which provides enhanced safety and reduces the likelihood of patient burns, which ensures that the temperature of an endoscopic device does not exceed a threshold temperature, which automatically controls the intensity of an illumination light based upon a sensed temperature of some portion of the endoscopic device, which employs temperature sensors that can fit within the available volume within typical endoscopic devices, which employs temperature sensors that do not create voltage, and therefore do not compromise patient safety, and which employs temperature sensors that do not require a mechanical wire connection, so that the endoscopic device may be readily autoclaved.

### Summary of the Invention

Accordingly, it is an object of the present invention to provide an endoscopic system which provides enhanced safety and reduces the likelihood of patient burns.

Another object of the present invention is to provide an endoscopic system having the above characteristics and which ensures that the temperature of an endoscopic device does not exceed a threshold temperature.

A further object of the present invention is to provide an endoscopic system having the above characteristics and which automatically controls the intensity of an illumination light based upon a sensed temperature of some portion of the endoscopic device.

Still another object of the present invention is to provide an endoscopic system having the above characteristics and which employs temperature sensors that can fit within the available volume within typical endoscopic devices.

Yet a further object of the present invention is to provide an endoscopic system having the above characteristics and which employs temperature sensors that do not create voltage, and therefore do not compromise patient safety.

Still yet a further object of the present invention is to provide an endoscopic system having the above characteristics and which employs temperature sensors that do not require a mechanical wire connection, so that the endoscopic device may be readily autoclaved.

These and other objects are achieved in accordance with one embodiment of the present invention by provision of an endoscopic system having an endoscopic device having a light guide passing therethrough to a distal end thereof, a light source in communication with the endoscopic device, the light source transmitting illumination light to the light guide of the endoscopic device, a light source controller in communication with the light source, the light source controller controlling an intensity of the illumination light transmitted to the light guide of the endoscopic device, and at least one temperature sensor, at least a portion of which is carried by the endoscopic device. The at least one temperature sensor senses a temperature of at least a portion of the endoscopic device and produces a signal indicative of the sensed temperature, the signal being transmitted to the light source controller. The light source controller varies the intensity of the illumination light transmitted to the light guide based at least in part upon the signal indicative of the sensed temperature.

In some embodiments, the light source controller varies the intensity of the illumination light transmitted to the light guide so as to maintain the sensed temperature below a threshold value. In certain of these embodiments, the light source controller reduces the intensity of the illumination light transmitted to the light guide if the sensed temperature is above the threshold value. In some embodiments, the endoscopic device is at least one of the following: a rigid endoscope, a semi-rigid endoscope and a flexible endoscope. In some embodiments, the at least one temperature sensor takes the form of a plurality of temperature sensors disposed along the endoscopic device.

In some embodiments, the at least one temperature sensor includes an optical fiber having a proximal end and a distal end, a member having a first end disposed adjacent the distal end of the optical fiber and a second end opposite the first end, the member being formed from a material with optical absorption/transmission properties that vary in a known relationship with respect to a temperature of the member, and a reflective surface disposed adjacent the second end of the member. In these embodiments, the light source supplies light energy to the proximal end of the optical fiber, and the light energy propagates to the distal end of the optical fiber, passes through the member from the first end to the second end thereof, is reflected by the reflective surface, passes through the member from the second end to the first end thereof, enters the distal end of the optical fiber, propagates to the proximal end of the optical fiber, and exits the proximal end of the optical fiber. The light energy is altered by the optical properties of the member as it passes through the member, and in a manner that is dependent upon the temperature of the member.

In accordance with another embodiment of the present invention, an endoscopic system includes an endoscopic device having at least one temperature sensor for sensing a temperature of at least a portion of the endoscopic device. The at least one temperature sensor includes an optical fiber having a proximal end and a distal end, a member having a first end disposed adjacent the distal end of the optical fiber and a second end opposite the first end, the member being formed from a material with optical absorption/transmission properties that vary in a known relationship with respect to a temperature of the member, and a reflective surface disposed adjacent the second end of the member. Light energy is supplied to the proximal end of the optical fiber, propagates to the distal end of the optical fiber, passes through the member from the first end to the second end thereof, is reflected by the reflective surface, passes through the member from the second end to the first end thereof, enters the distal end of the optical fiber, propagates to the proximal end of the optical fiber, and exits the proximal end of the optical fiber. The light energy is altered by the optical properties of the member as it passes through the member, and in a manner that is dependent upon the temperature of the member.

In some embodiments, the at least one temperature sensor further includes a light energy analyzer for analyzing the properties of the light energy exiting the proximal end of the optical fiber, and for generating a signal indicative thereof. In certain of these embodiments, the light energy analyzer is a spectrophotometer. In certain embodiments, the at least one temperature sensor further includes a temperature analyzer for determining the temperature of the member based at least in part upon the signal indicative of the properties of the light energy exiting the proximal end of the optical fiber, and based at least in part upon the known relationship between the optical absorption/transmission properties of the member and the temperature of the member. In certain of these embodiments, the at least one temperature sensor further includes a temperature display for displaying the temperature of the member.

In some embodiments, the endoscopic system further includes a light source in communication with the endoscopic device, the light source transmitting illumination light to a light guide of the endoscopic device, and a light source controller in communication with the light source, the light source controller controlling an intensity of the illumination light transmitted to the light guide of the endoscopic device. In these embodiments, the light source controller varies the intensity of the illumination light transmitted to the light guide based at least in part upon a temperature sensed by the at least one temperature sensor.

In accordance with a further embodiment of the present invention, an endoscopic system includes an endoscopic device having a light guide passing therethrough to a distal end thereof, a light source in communication with the endoscopic device, the light source transmitting illumination light to the light guide of the endoscopic device, a light source controller in communication with the light source, the light source controller controlling an intensity of the illumination light transmitted to the light guide of the endoscopic device, and at least one temperature sensor. The temperature sensor includes an optical fiber having a proximal end and a distal end, a member having a first end disposed adjacent the distal end of the optical fiber and a second end opposite the first end, the member being formed from a material with optical absorption/transmission properties that vary in a known relationship with respect to a temperature of the member, and a reflective surface disposed adjacent the second end of the member. Light energy is supplied to the proximal end of the optical fiber, propagates to the distal end of the optical fiber, passes through the member from the first end to the second end thereof, is reflected by the reflective surface, passes through the member from the second end to the first end thereof, enters the distal end of the optical fiber, propagates to the proximal end of the optical fiber, and exits the proximal end of the optical fiber. The light energy is altered by the optical properties of the member as it passes through the member, and in a manner that is dependent upon the temperature of the member. The endoscopic system further includes a light energy analyzer for analyzing the properties of the light energy exiting the proximal end of the optical fiber, and for generating a signal indicative thereof, and a temperature analyzer for determining the temperature of the member based at least in part upon the signal indicative of the properties of the light energy exiting the proximal end of the optical fiber, and based at least in part upon the known relationship between the optical absorption/transmission properties of the member and the temperature of the member. The temperature analyzer generates and transmits a signal indicative of the determined temperature of the member to the light source controller, and the light source controller varies the intensity of the illumination light transmitted to the light guide based at least in part upon the signal indicative of the determined temperature of the member.

In accordance with still another embodiment of the present invention, a method of controlling an endoscopic system includes the steps of: (i) providing an endoscopic device having a light guide passing therethrough to a distal end thereof; (ii) transmitting illumination light to the light guide of the endoscopic device using a light source in communication with the endoscopic device; (iii) sensing a temperature of at least a portion of the endoscopic device and producing a signal indicative of the sensed temperature using at least one temperature sensor, at least a portion of which is carried by the endoscopic device; (iv) transmitting the signal indicative of the sensed temperature to a light source controller; and (v) varying the intensity of the illumination light transmitted to the light guide automatically, using the light source controller, based at least in part upon the signal indicative of the sensed temperature.

In some embodiments, the varying step (v) involves the step of varying the intensity of the illumination light transmitted to the light guide automatically, using the light source controller, based at least in part upon the signal indicative of the sensed temperature so as to maintain the sensed temperature below a threshold value. In certain of these embodiments, the varying step (v) involves the step of reducing the intensity of the illumination light transmitted to the light guide automatically, using the light source controller, if the signal indicative of the sensed temperature indicates that the sensed temperature is above the threshold value. In some embodiments, the at least one temperature sensor takes the form of a plurality of temperature sensors disposed along the endoscopic device.

In some embodiments, the sensing step (iii) involves the steps of: (a) providing an optical fiber having a proximal end and a distal end; (b) disposing a member with a first end thereof adjacent the distal end of the optical fiber and a second end opposite the first end, the member formed from a material with optical absorption/transmission properties that vary in a known relationship with respect to a temperature of the member; (c) disposing a reflective surface adjacent the second end of the member; and (d) supplying light energy to the proximal end of the optical fiber, propagating the light energy to the distal end of the optical fiber, passing the light energy through the member from the first end to the second end thereof, reflecting the light energy with the reflective surface, passing the light energy through the member from the second end to the first end thereof, causing the light energy to enter the distal end of the optical fiber, propagating the light energy to the proximal end of the optical fiber, and causing the light energy to exit the proximal end of the optical fiber. In these embodiments, the light energy is altered by the optical properties of the member as it passes through the member, and in a manner that is dependent upon the temperature of the member.

In accordance with still a further embodiment of the present invention, a method for sensing a temperature of at least a portion of an endoscopic device includes the steps of: (i) providing an optical fiber having a proximal end and a distal end; (ii) disposing a member with a first end disposed adjacent the distal end of the optical fiber and a second end opposite the first end, the member formed from a material with optical absorption/transmission properties that vary in a known relationship with respect to a temperature of the member; (iii) disposing a reflective surface adjacent the second end of the member; and (iv) supplying light energy to the proximal end of the optical fiber, propagating the light energy to the distal end of the optical fiber, passing the light energy through the member from the first end to the second end thereof, reflecting the light energy with the reflective surface, passing the light energy through the member from the second end to the first end thereof, causing the light energy to enter the distal end of the optical fiber, propagating the light energy to the proximal end of the optical fiber, and causing the light energy to exit the proximal end of the optical fiber. The light energy is altered by the optical properties of the member as it passes through the member, and in a manner that is dependent upon the temperature of the member.

In some embodiments, the method for sensing a temperature of at least a portion of an endoscopic device further includes the step of: (v) analyzing the properties of the light energy exiting the proximal end of the optical fiber, and generating a signal indicative thereof. In certain of these embodiments, the analyzing step (v) is performed using a spectrophotometer. In certain embodiments, the method for sensing a temperature of at least a portion of an endoscopic device further includes the step of: (vi) determining the temperature of the member based at least in part upon the signal indicative of the properties of the light energy exiting the proximal end of the optical fiber, and based at least in part upon the known relationship between the optical absorption/transmission properties of the member and the temperature of the member. In certain of these embodiments, the method for sensing a temperature of at least a portion of an endoscopic device further includes the step of: (vii) displaying the temperature of the member.

In some embodiments, the method for sensing a temperature of at least a portion of an endoscopic device further includes the step of: (v) using the sensed temperature to vary an intensity of illumination light supplied to the endoscopic device.

In accordance with yet another embodiment of the present invention, a method of controlling an endoscopic system includes the steps of: (i) providing an endoscopic device having a light guide passing therethrough to a distal end thereof; (ii) transmitting illumination light to the light guide of the endoscopic device using a light source in communication with the endoscopic device; (iii) providing an optical fiber having a proximal end and a distal end; (iv) disposing a member with a first end disposed adjacent the distal end of the optical fiber and a second end opposite the first end, the member formed from a material with optical absorption/transmission properties that vary in a known relationship with respect to a temperature of the member; (v) disposing a reflective surface adjacent the second end of the member; (vi) supplying light energy to the proximal end of the optical fiber, propagating the light energy to the distal end of the optical fiber, passing the light energy through the member from the first end to the second end thereof, reflecting the light energy with the reflective surface, passing the light energy through the member from the second end to the first end thereof, causing the light energy to enter the distal end of the optical fiber, propagating the light energy to the proximal end of the optical fiber, and causing the light energy to exit the proximal end of the optical fiber, wherein the light energy is altered by the optical properties of the member as it passes through the member, and in a manner that is dependent upon the temperature of the member; (vii) analyzing the properties of the light energy exiting the proximal end of the optical fiber, and generating a signal indicative thereof; (viii) determining the temperature of the member based at least in part upon the signal indicative of the properties of the light energy exiting the proximal end of the optical fiber, and based at least in part upon the known relationship between the optical absorption/transmission properties of the member and the temperature of the member; and (ix) varying the intensity of the illumination light transmitted to the light guide automatically, using a light source controller, based at least in part upon the determined temperature of the member.

The invention and its particular features and advantages will become more apparent from the following detailed description considered with reference to the accompanying drawings.

### Brief Description of the Drawings

**Figure 1** is a schematic view of an endoscopic system in accordance with an embodiment of the present invention; and

Figure 2 is an enlarged, schematic view of an embodiment of a sensor employed by the endoscopic system of Figure 1.

### Detailed Description of an Embodiment of the Invention

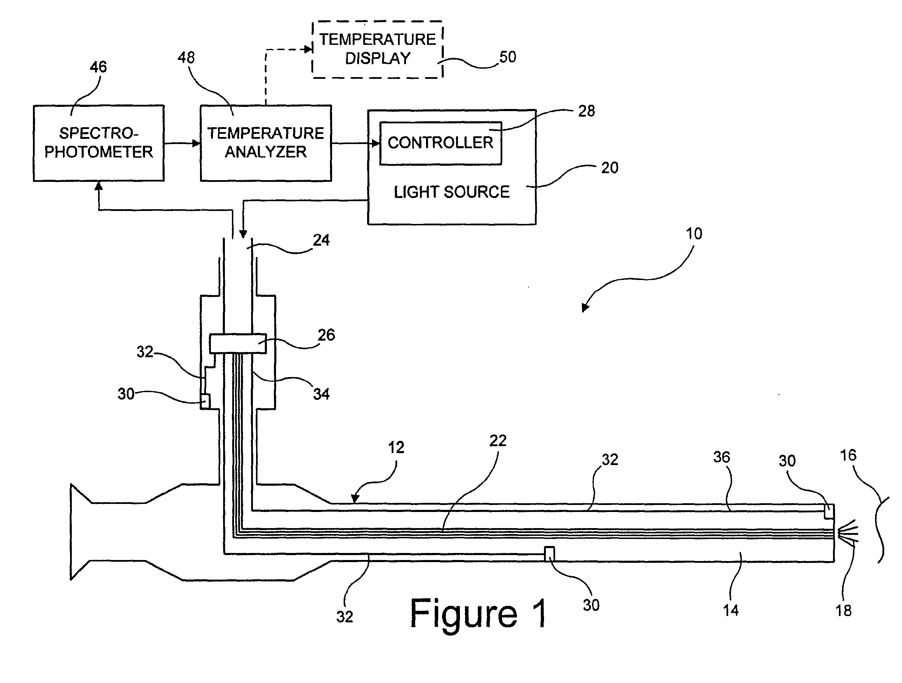

Referring first to Figure 1, an endoscopic system 10 in accordance with an exemplary embodiment of the present invention is shown. Endoscopic system 10 includes an endoscopic device 12, which may comprise a conventional endoscope with an eyepiece and an optical transmission means, such as a lens system or a fiber optic system, an electronic endoscope having a distally disposed imaging unit or camera head mounted at the proximal end to produce video images, or some other type of image viewing system. In some embodiments, the imaging unit includes an image sensor (e.g., CCD, CMOS). The endoscopic system 10 may further include an imaging controller in communication with the imaging unit for controlling and varying the image amplification, gain, and/or exposure time of the imaging unit. However, because numerous types of image capture/viewing systems are well known in the art, and because the present invention is concerned with the illumination system, rather than the image capture/viewing system, the numerous image capture/viewing systems in connection with which the present invention may be used are not described herein and the elements thereof are not shown in the Figures for the sake of clarity. Moreover, it should be noted that the present invention may be used in connection with substantially any type of known or later developed endoscopic devices, such as, but not limited to, rigid endoscopes, semi-rigid endoscopes, and flexible endoscopes.

Endoscopic device 12 includes a distal end 14 which during use is typically inserted into an orifice or body cavity and is directed at tissue 16 to inspect the tissue 16. As is known, there is typically very little light within the orifice or body cavity, such that illumination light 18 is required to be provided in order to illuminate tissue 16 for viewing. Typically, this illumination light 18 is provided by a high intensity light source 20 and passed to the distal end 14 of endoscopic device 12 through a light guide 22 that passes through the endoscopic device 12 to the distal end 14.

The light guide 22 may take the form of, for example, a fiber optic bundle coupled to a light cable 24 supplied light energy from light source 20 by way of an optical coupler 26 or the like. Of course, light guide 22 may take other forms.

Light source 20 may comprise any of numerous types of known or yet to be developed light sources. One type of known light source is a high intensity light source that utilizes an incandescent bulb (such as a xenon bulb, or other type), driven by an amplifier, which in turn is controlled by output control circuitry, to set the light intensity level of the light source. Of course, other types of light source intensity output control are known within the art, such as mechanical diaphragm or iris, liquid crystal shutter, rotary reed or slot devices, and the like. These various types of light source output controls may be utilized within the system of the present invention. All that is required is that the light source 20 for use in accordance with the present invention have a controller 28 that is capable of automatically controlling an intensity of illumination light 18 transmitted to light guide 22 of endoscopic device 12 in response to input signals (as described in more detail below). The light source 20 may also comprise semiconductor element light sources such as light emitting diodes ("LED") and/or diode-lasers (e.g., with fast switching characteristics).

Endoscopic device 12 includes at least one, but preferably a plurality of, temperature sensors 30 associated therewith sensing a temperature of at least a portion of endoscopic device 12 and producing a signal indicative of the sensed temperature. Preferably, temperature sensors 30 are disposed within endoscopic device, but if desired, they may be carried on an external surface thereof. It is also preferable that temperature sensors 30 be spaced apart at various locations along endoscopic device 12 so as to provide temperature readings at various locations thereof to ensure that the desired maximum temperature is not exceeded locally at any location thereof.

The signals indicative of the sensed temperature produced by temperature sensors 30 are transmitted to light source controller 28, which varies the intensity of illumination light 18 transmitted to light guide 22 based at least in part upon the signal indicative of the sensed temperature. More specifically, light source controller 28 varies the intensity of illumination light 18 transmitted to light guide 22 so as to maintain the sensed temperature below a threshold value. As discussed above, the threshold value is in some circumstances 50 °C, but some other threshold value may be appropriate or dictated by appropriate standards. In some embodiments, the signals indicative of the sensed temperature are also transmitted to the imaging controller to synchronize switching of the light source 20 with the imaging unit and/or video signal. The imaging controller may further receive signals from the light source controller 28 to adjust or switch the imaging unit in synchrony with the light source 20.

Any of numerous algorithms may be employed by light source controller 28 to vary the intensity of illumination light 18 based at least in part upon the signal indicative of the sensed temperature. One simple algorithm employed may be to reduce the intensity of illumination light 18 transmitted to light guide 22 if the temperature sensed by any of temperature sensors 30 is above the threshold value (e.g., 50 °C), and then to increase the intensity of illumination light 18 again if the temperature sensed by all of temperature sensors 30 falls below another value (e.g., the threshold value minus 3 °C, or 47 °C). Of course, one skilled in the art could easily and routinely program light source controller 28 with other control algorithms.

In some embodiments, the imaging unit is adjusted is synchrony with the light source 20. For example, the image amplification and/or exposure time of the imaging unit may be increased as the intensity of the illumination light 18 is decreased. In other embodiments, the process of pixel binning is used to group several pixels in proximity to one another as the intensity of the illumination light 18 is decreased. Pixel binning decreases image resolution but increases image brightness. In further embodiments, the light source 20 is pulsed by the light source controller 28 such that only each first half image of the video stream (e.g., PAL, NTSC) is illuminated and displayed twice by means of an image processing and display unit. This reduction generates only half of the light energy. The video output frequency remains the same and only the resolution is reduced.

In some embodiments, the endoscopic system further includes an alarm that is activated in response to signals indicative of the sensed temperature. For example, the alarm may be activated when the temperature sensed by any of temperature sensors 30 is above the threshold value. The system may also include an integrated cooling system (e.g., heat pipe, Peltier-element) that is activated when the temperature sensed by any of temperature sensors 30 is above the threshold value.

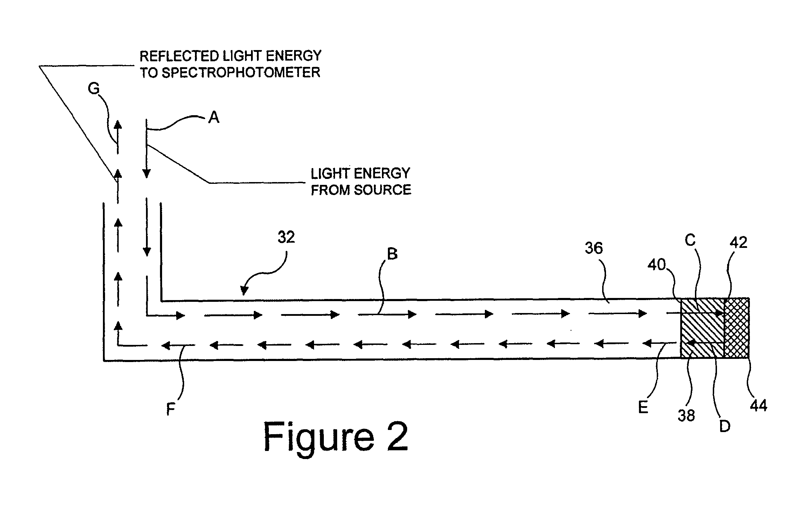

As discussed above in greater detail, known temperature sensors, such as thermocouples, suffer from a number of disadvantages which typically make them unsuitable for use in connection with endoscopic system 10 in accordance with the present invention. Referring now to Figure 2 in addition to Figure 1, temperature sensors 30 in accordance with another aspect of the present invention will be discussed in more detail.

Each sensor 30 includes an optical fiber 32 having a proximal end 34, which is supplied with light energy, and a distal end 36. The light energy supplied to proximal end 34 may be supplied by light source 20, through, for example, optical coupler 26, or may be supplied by some other source. It should be understood that optical fiber 32 may comprise a single strand or a plurality of strands.

Each sensor 30 also includes a member 38 having a first end 40 disposed adjacent distal end 36 of optical fiber 32 and a second end 42 opposite the first end 40. Member 38 comprises a material with optical absorption/transmission properties that vary in a known relationship with respect to a temperature thereof. For example, member 38 may be formed from a material that allows different frequencies of light energy to pass therethrough at different temperatures thereof, different amounts of light energy to pass therethrough at different temperatures thereof, etc. The particular optical properties that vary with temperature are not important, so long as the relationship between the variance of the optical properties and temperature is known. One example of a material from which member 38 may be formed is boresilicate glass doped with neodymium, which material allows varying frequencies of light energy to pass therethrough at varying temperatures, the particular relationship therebetween having been well-documented.

A reflective surface 44 is disposed adjacent second end 42 of member 38, which acts to reflect a significant portion, and preferably substantially all, light energy striking reflective surface 44. Reflective surface 44 may comprise a mirror or any of numerous other reflective elements/materials known or later developed. Reflective surface 44 may comprise a separate element attached to second end 42 of member 38, may be painted, metallized, or otherwise applied directly onto second end 42 of member 38, may be positioned adjacent second end 42 of member 38, etc.

The light energy supplied to the proximal end 34 of optical fiber 32 (indicated by A), propagates to distal end 36 of optical fiber 32 (indicated by B), passes through member 38 from first end 40 to second end 42 thereof (indicated by C), is reflected by reflective surface 44, passes through member 38 from second end 42 to first end 40 thereof (indicated by D), enters distal end 36 of optical fiber 32 (indicated by E), propagates to proximal end 34 of optical fiber 32 (indicated by F), and exits proximal end 34 of optical fiber 32 (indicated by G). As indicated by the difference in size between arrows A, B representing the light energy before passing through member 38 and arrows E, F, G representing the light energy after passing through member 38, the light energy is altered by the optical properties of member 38 as it passes therethrough, and in a manner that is dependent upon the temperature of member 38, as discussed above.

The properties of the light energy exiting proximal end 34 of optical fiber 32 (indicated by G) is analyzed using a light energy analyzer 46, which generates a signal indicative of such properties. In the illustrated embodiment, light energy analyzer 46 comprises a spectrophotometer. Thus, in the case where member 38 is formed from a material which allows varying frequencies of light energy to pass therethrough at varying temperatures, the spectrophotometer could be used to measure the frequencies of the light energy exiting proximal end 34 of optical fiber 32 (indicated by G) and to generate a signal indicative of these frequencies.

A temperature analyzer 48 receives the signal indicative of the properties of the light energy exiting proximal end 34 of optical fiber 32 (indicated by G) generated by light energy analyzer 46, and determines the temperature of member 38 based at least in part upon the this signal, and based at least in part upon the known relationship between the optical absorption/transmission properties of member 38 and the temperature of member 38. Thus, temperature analyzer 48 has stored thereon, or otherwise has access to, data indicative of the known relationship between the variance of the optical properties and temperature for the material from which member 38 is made.

A temperature display 50 may optionally (indicated by dashed lines) be provided for displaying the sensed temperature of member 38. When multiple members 38 are provided, temperature display 50 may display all of the sensed temperatures, or may display only some of the sensed temperatures (e.g., the highest temperature).

Although light energy analyzer 46, temperature analyzer 48, temperature display 50 and controller 28 of light source 20 are shown as separate elements in Figure 1, any two or more of them may be combined into one or more integrated units.

The present invention, therefore, provides an endoscopic system which provides enhanced safety and reduces the likelihood of patient burns, which ensures that the temperature of an endoscopic device does not exceed a threshold temperature, which automatically controls the intensity of an illumination light based upon a sensed temperature of some portion of the endoscopic device, which employs temperature sensors that can fit within the available volume within typical endoscopic devices, which employs temperature sensors that do not create voltage, and therefore do not compromise patient safety, and which employs temperature sensors that do not require a mechanical wire connection, so that the endoscopic device may be readily autoclaved.

Although the invention has been described with reference to a particular arrangement of parts, features and the like, these are not intended to exhaust all possible arrangements or features, and indeed many other modifications and variations will be ascertainable to those of skill in the art.

## Claims

1. An endoscopic system comprising:
an endoscopic device having a light guide passing therethrough to a distal end thereof;
a light source in communication with said endoscopic device, said light source transmitting illumination light to the light guide of said endoscopic device;
a light source controller in communication with said light source, said light source controller controlling an intensity of the illumination light transmitted to the light guide of said endoscopic device;
at least one temperature sensor, at least a portion of which is carried by said endoscopic device, said at least one temperature sensor sensing a temperature of at least a portion of said endoscopic device and producing a signal indicative of the sensed temperature, the signal being transmitted to said light source controller; and
wherein said light source controller varies the intensity of the illumination light transmitted to the light guide based at least in part upon the signal indicative of the sensed temperature.

2. The endoscopic system of Claim 1 wherein said light source controller varies the intensity of the illumination light transmitted to the light guide so as to maintain the sensed temperature below a threshold value.

3. The endoscopic system of Claim 2 wherein said light source controller reduces the intensity of the illumination light transmitted to the light guide if the sensed temperature is above the threshold value.

4. The endoscopic system of Claim 1 wherein said endoscopic device comprises at least one of the following: a rigid endoscope, a semi-rigid endoscope and a flexible endoscope.

5. The endoscopic system of Claim 1 wherein said at least one temperature sensor comprises a plurality of temperature sensors disposed along said endoscopic device.

6. The endoscopic system of Claim 1 wherein said at least one temperature sensor comprises:
an optical fiber having a proximal end and a distal end;
a member having a first end disposed adjacent the distal end of said optical fiber and a second end opposite the first end, said member comprising a material with optical absorption/transmission properties that vary in a known relationship with respect to a temperature of said member;
a reflective surface disposed adjacent the second end of said member;
wherein said light source supplies light energy to the proximal end of said optical fiber, and wherein the light energy propagates to the distal end of said optical fiber, passes through said member from the first end to the second end thereof, is reflected by said reflective surface, passes through said member from the second end to the first end thereof, enters the distal end of said optical fiber, propagates to the proximal end of said optical fiber, and exits the proximal end of said optical fiber; and
wherein the light energy is altered by the optical properties of said member as it passes through said member, and in a manner that is dependent upon the temperature of said member.

7. The endoscopic system according to claim 1, further comprising:
an imaging unit comprising an image sensor;
an imaging controller for varying an image amplification and an exposure time of the imaging unit.

8. The endoscopic system according to claim 7, wherein said imaging controller varies at least one of the image amplification and the exposure time based at least in part upon the signal indicative of the sensed temperature.

9. The endoscopic system according to claim 7, wherein the imaging controller varies at least one of the image amplification and exposure time in synchrony with said light source controller varying the intensity of the illumination light.

10. The endoscopic system according to claim 1, wherein the light source includes at least one of a light emitting diode and a diode-laser.

11. The endoscopic system according to claim 1, further comprising:
a cooling system, wherein said cooling system is activated based at least in part upon the signal indicative of the sensed temperature.

12. The endoscopic system according to claim 1, further comprising:
an alarm, wherein said alarm is activated based at least in part upon the signal indicative of the sensed temperature.

13. An endoscopic system comprising an endoscopic device having at least one temperature sensor for sensing a temperature of at least a portion of the endoscopic device, the at least one temperature sensor comprising:
an optical fiber having a proximal end and a distal end;
a member having a first end disposed adjacent the distal end of said optical fiber and a second end opposite the first end, said member comprising a material with optical absorption/transmission properties that vary in a known relationship with respect to a temperature of said member;
a reflective surface disposed adjacent the second end of said member;
wherein light energy is supplied to the proximal end of said optical fiber, propagates to the distal end of said optical fiber, passes through said member from the first end to the second end thereof, is reflected by said reflective surface, passes through said member from the second end to the first end thereof, enters the distal end of said optical fiber, propagates to the proximal end of said optical fiber, and exits the proximal end of said optical fiber; and
wherein the light energy is altered by the optical properties of said member as it passes through said member, and in a manner that is dependent upon the temperature of said member.

14. The endoscopic system of Claim 13, wherein the at least one temperature sensor further comprises a light energy analyzer for analyzing the properties of the light energy exiting the proximal end of said optical fiber, and for generating a signal indicative thereof.

15. The endoscopic system of Claim 14, wherein the light energy analyzer comprises a spectrophotometer.

16. The endoscopic system of Claim 14, wherein the at least one temperature sensor further comprises a temperature analyzer for determining the temperature of said member based at least in part upon the signal indicative of the properties of the light energy exiting the proximal end of said optical fiber, and based at least in part upon the known relationship between the optical absorption/transmission properties of said member and the temperature of said member.

17. The endoscopic system of Claim 16, wherein the at least one temperature sensor further comprises a temperature display for displaying the temperature of said member.

18. The endoscopic system of Claim 13 further comprising:
a light source in communication with said endoscopic device, said light source transmitting illumination light to a light guide of said endoscopic device;
a light source controller in communication with said light source, said light source controller controlling an intensity of the illumination light transmitted to the light guide of said endoscopic device; and
wherein said light source controller varies the intensity of the illumination light transmitted to the light guide based at least in part upon a temperature sensed by the at least one temperature sensor.

19. An endoscopic system comprising:
an endoscopic device having a light guide passing therethrough to a distal end thereof;
a light source in communication with said endoscopic device, said light source transmitting illumination light to the light guide of said endoscopic device;
a light source controller in communication with said light source, said light source controller controlling an intensity of the illumination light transmitted to the light guide of said endoscopic device;
at least one temperature sensor comprising:
an optical fiber having a proximal end and a distal end;
a member having a first end disposed adjacent the distal end of said optical fiber and a second end opposite the first end, said member comprising a material with optical absorption/transmission properties that vary in a known relationship with respect to a temperature of said member;
a reflective surface disposed adjacent the second end of said member;
wherein light energy is supplied to the proximal end of said optical fiber, propagates to the distal end of said optical fiber, passes through said member from the first end to the second end thereof, is reflected by said reflective surface, passes through said member from the second end to the first end thereof, enters the distal end of said optical fiber, propagates to the proximal end of said optical fiber, and exits the proximal end of said optical fiber;
wherein the light energy is altered by the optical properties of said member as it passes through said member, and in a manner that is dependent upon the temperature of said member;
a light energy analyzer for analyzing the properties of the light energy exiting the proximal end of said optical fiber, and for generating a signal indicative thereof; and
a temperature analyzer for determining the temperature of said member based at least in part upon the signal indicative of the properties of the light energy exiting the proximal end of said optical fiber, and based at least in part upon the known relationship between the optical absorption/transmission properties of said member and the temperature of said member, said temperature analyzer generating and transmitting a signal indicative of the determined temperature of said member to said light source controller; and
wherein said light source controller varies the intensity of the illumination light transmitted to the light guide based at least in part upon the signal indicative of the determined temperature of said member.

20. A method of controlling an endoscopic system comprising the steps of:
providing an endoscopic device having a light guide passing therethrough to a distal end thereof;
transmitting illumination light to the light guide of the endoscopic device using a light source in communication with the endoscopic device;
sensing a temperature of at least a portion of the endoscopic device and producing a signal indicative of the sensed temperature using at least one temperature sensor, at least a portion of which is carried by the endoscopic device;
transmitting the signal indicative of the sensed temperature to a light source controller; and
varying the intensity of the illumination light transmitted to the light guide automatically, using the light source controller, based at least in part upon the signal indicative of the sensed temperature.

21. The method of controlling an endoscopic system of Claim 20 wherein said varying step comprises the step of varying the intensity of the illumination light transmitted to the light guide automatically, using the light source controller, based at least in part upon the signal indicative of the sensed temperature so as to maintain the sensed temperature below a threshold value.

22. The method of controlling an endoscopic system of Claim 21 wherein said varying step comprises the step of reducing the intensity of the illumination light transmitted to the light guide automatically, using the light source controller, if the signal indicative of the sensed temperature indicates that the sensed temperature is above the threshold value.

23. The method according to claim 22, further comprising the step of:
grouping two or more pixels of a received image via pixel binning to increase brightness of the received image when the intensity of the illumination light is reduced.

24. The method of controlling an endoscopic system of Claim 14 wherein the at least one temperature sensor comprises a plurality of temperature sensors disposed along the endoscopic device.

25. The method of controlling an endoscopic system of Claim 20 wherein said sensing step comprises the steps of:
providing an optical fiber having a proximal end and a distal end;
disposing a member with a first end thereof adjacent the distal end of the optical fiber and a second end opposite the first end, the member comprising a material with optical absorption/transmission properties that vary in a known relationship with respect to a temperature of the member;
disposing a reflective surface adjacent the second end of the member;
supplying light energy to the proximal end of the optical fiber, propagating the light energy to the distal end of the optical fiber, passing the light energy through the member from the first end to the second end thereof, reflecting the light energy with the reflective surface, passing the light energy through the member from the second end to the first end thereof, causing the light energy to enter the distal end of the optical fiber, propagating the light energy to the proximal end of the optical fiber, and causing the light energy to exit the proximal end of the optical fiber; and
wherein the light energy is altered by the optical properties of the member as it passes through the member, and in a manner that is dependent upon the temperature of the member.

26. The method according to claim 20:
wherein the endoscopic device includes an imaging unit; and
wherein the method further comprises the step of varying at least one of an image amplification and an exposure time of an imaging unit based at least in part upon the signal indicative of the sensed temperature.

27. The method according to claim 20:
wherein the endoscopic device includes an imaging unit; and
wherein the method further comprises the step of varying at least one of an image amplification and an exposure time of an imaging unit in synchrony with the varying of the intensity of the illumination light.

28. A method for sensing a temperature of at least a portion of an endoscopic device comprising the steps of:
providing an optical fiber having a proximal end and a distal end;
disposing a member with a first end disposed adjacent the distal end of the optical fiber and a second end opposite the first end, the member comprising a material with optical absorption/transmission properties that vary in a known relationship with respect to a temperature of the member;
disposing a reflective surface adjacent the second end of the member;
supplying light energy to the proximal end of the optical fiber, propagating the light energy to the distal end of the optical fiber, passing the light energy through the member from the first end to the second end thereof, reflecting the light energy with the reflective surface, passing the light energy through the member from the second end to the first end thereof, causing the light energy to enter the distal end of the optical fiber, propagating the light energy to the proximal end of the optical fiber, and causing the light energy to exit the proximal end of the optical fiber; and
wherein the light energy is altered by the optical properties of the member as it passes through the member, and in a manner that is dependent upon the temperature of the member.

29. The method for sensing a temperature of at least a portion of an endoscopic device of Claim 28, further comprising the step of analyzing the properties of the light energy exiting the proximal end of the optical fiber, and generating a signal indicative thereof.

30. The method for sensing a temperature of at least a portion of an endoscopic device of Claim 29, wherein said analyzing step is performed using a spectrophotometer.

31. The method for sensing a temperature of at least a portion of an endoscopic device of Claim 29, further comprising the step of determining the temperature of the member based at least in part upon the signal indicative of the properties of the light energy exiting the proximal end of the optical fiber, and based at least in part upon the known relationship between the optical absorption/transmission properties of the member and the temperature of the member.

32. The method for sensing a temperature of at least a portion of an endoscopic device of Claim 31, further comprising the step of displaying the temperature of the member.

33. The method for sensing a temperature of at least a portion of an endoscopic device of Claim 28 further comprising the step of using the sensed temperature to vary an intensity of illumination light supplied to the endoscopic device.

34. A method of controlling an endoscopic system comprising the steps of:
providing an endoscopic device having a light guide passing therethrough to a distal end thereof;
transmitting illumination light to the light guide of the endoscopic device using a light source in communication with the endoscopic device;
providing an optical fiber having a proximal end and a distal end;
disposing a member with a first end disposed adjacent the distal end of the optical fiber and a second end opposite the first end, the member comprising a material with optical absorption/transmission properties that vary in a known relationship with respect to a temperature of the member;
disposing a reflective surface adjacent the second end of the member;
supplying light energy to the proximal end of the optical fiber, propagating the light energy to the distal end of the optical fiber, passing the light energy through the member from the first end to the second end thereof, reflecting the light energy with the reflective surface, passing the light energy through the member from the second end to the first end thereof, causing the light energy to enter the distal end of the optical fiber, propagating the light energy to the proximal end of the optical fiber, and causing the light energy to exit the proximal end of the optical fiber;
wherein the light energy is altered by the optical properties of the member as it passes through the member, and in a manner that is dependent upon the temperature of the member;
analyzing the properties of the light energy exiting the proximal end of the optical fiber, and generating a signal indicative thereof;
determining the temperature of the member based at least in part upon the signal indicative of the properties of the light energy exiting the proximal end of the optical fiber, and based at least in part upon the known relationship between the optical absorption/transmission properties of the member and the temperature of the member; and
varying the intensity of the illumination light transmitted to the light guide automatically, using a light source controller, based at least in part upon the determined temperature of the member.
